# FASCICULE DE BREVET EUROPEEN

(11) **EP 0 592 636 B1**
(45) Date de publication et mention de la délivrance du brevet: **25.11.1998**
(21) Numéro de dépôt: 93908990.0
(22) Date de dépôt: 02.04.1993
(51) Int. Cl.: C12N 7/00, C12N 5/08

(54) **PROCEDE D'OBTENTION D'UNE CULTURE CELLULAIRE VIABLE INFECTEE PAR UN VIRUS ASSOCIE A LA SCLEROSE EN PLAQUES**
VERFAHREN ZUR HERSTELLUNG VON LEBENDEN ZELLKULTUREN, DIE DURCH EINEN MULTIPLE SKLEROSE ASSOZIIERTEN VIRUS INFIZIERT SIND
PROCESS FOR THE PRODUCTION OF A VIABLE CELL CULTURE INFECTED BY A MULTIPLE SCLEROSIS ASSOCIATED VIRUS

(30) Priorité: 03.04.1992 FR 9204322; 03.11.1992 FR 9213447
(43) Date de publication de la demande: 20.04.1994
(73) Titulaire: BIO MERIEUX, 69280 Marcy l'Etoile (FR); UNIVERSITE JOSEPH FOURIER (GRENOBLE 1), F-38041 Grenoble Cédex 09 (FR)
(72) Inventeur: PERRON, Hervé, F-38100 Grenoble (FR); SEIGNEURIN, Jean-Marie, F-38190 Bernin Cédex 49 (FR)
(74) Mandataire: Guerre, Dominique
(86) Numéro de dépôt international: FR9300337
(87) Numéro de publication internationale: WO9320189

(56) Documents cités:
- BIOLOGICAL ABSTRACTS vol. 89, no. 9 , 1 Mai 1990, Philadelphia, PA, US; abstract no. 94670, H. PERRON ET AL. 'LEPTOMENINGEAL CELL LINE FROM MULTIPLE SCLEROSIS WITH REVERSE TRANSCRIPTASE ACTIVITY AND VIRAL PARTICLES.' page AB-568 ; cité dans la demande
- THE LANCET vol. 337, no. 8745, 6 Avril 1991, LONDON, GB pages 862 - 863 H. PERRON ET AL. 'ISOLATION OF RETROVIRUS FROM PATIENTS WITH MULTIPLE SCLEROSIS' cité dans la demande
- BIOLOGICAL ABSTRACTS vol. 83, no. 7 , 1987, Philadelphia, PA, US; abstract no. 71304, C. BOSGIRAUD ET AL. 'ULTRASTRUCTURAL STUDY ON VISNA VIRUS IN SHEEP PLEXUS CHOROID CELLS.' page AB-1195 ;
- AMERICAN TYPE CULTURE COLLECTION CATALOGUE OF CELL LINES AND HYBRIDOMAS, 6TH EDITION 1988, ROCKVILLE, MD, US R. HAY ET AL., EDITORS

## Description

La présente invention a trait à l'obtention et au maintien d'une culture ou lignée cellulaire viable, infectée par le virus associé à la sclérose en plaques (SEP). Une telle culture présente en effet l'intérêt de constituer un matériel biologique, exprimant l'activité du virus associé à la SEP, et pouvant de ce fait servir à différentes fins expérimentales, notamment à des tests d'identification et caractérisation du virus, mais aussi à des fins cliniques ou thérapeutiques.

Par "culture viable", on entend toute culture maintenant en vie les cellules infectées, de manière à exprimer le virus associé à la SEP, et préservant le pouvoir mitotique desdites cellules, notamment lors d'une culture par passages successifs de ces dernières.

La présente invention a pour objet un procédé pour la culture in vitro de cellules infectées par un virus présent chez des individus atteints de sclérose en plaques.

La sclérose en plaques (SEP) est une maladie démyélinisante du système nerveux central (SNC) que l'on suspecte depuis plusieurs années être associée à un virus, bien que l'agent causal n'ait pas encore été déterminé avec certitude.

De nombreux travaux ont étayé cette hypothèse d'une étiologie virale de la maladie, mais aucun des virus connus testés ne s'est avéré être l'agent causal recherché.

Par la suite, l'observation chez des patients atteints de sclérose en plaques de phénomènes assimilables à une réaction d'auto-immunité a conduit à une hypothèse étiologique auto-immune "essentielle" (Lisak R.P., Zweiman B. New Engl. J. Med. 1977; 297, 850-853, et Lassmann H. et Wisniewski H.M. Arch. Neurol. 1979; 36, 490-497). Cependant, cette auto-immunité dirigée contre certains composants du système nerveux central s'est révélée peu spécifique de la SEP et fréquente dans des inflammations du SNC, associées ou non à une infection, comme cela a été montré par Hirayama M. et al. (Neurology 1986 ; 36, 276-8) Kenneth G. Warren et al. (Annals of Neurology 1986 ; 20, 20-25), Suzumura A. et al. (Journal of Neuroimmunology 1986 ; 11, 137-47) et Tourtelotte W. et al. (Journal of Neurochemistry 1986 ; 46, 1086-93). De plus, comme l'a fait remarquer E.J. Field (The Lancet 1989 ; 1, 1272), aucune des thérapeutiques immunosuppressives n'a permis d'obtenir des résultats décisifs contre la SEP.

Une hypothèse a été émise selon laquelle un rétrovirus serait à l'origine de la maladie. La découverte par A. Gessain et al. (J. Infect. Disease 1988 ; 1226-1234), de syndromes neurologiques associés au virus HTLV-1, connu à l'origine comme agent de leucémies T de l'adulte, a conduit de nombreux auteurs tels que H. Koprowski et al. (Nature 1985 ; 318, 154), M. Ohta et al. (J. Immunol. 1986 ; 137, 3440), E.P. Reddy et al. (Science 1989 ; 243, 529), S.J. Greenberg et al. (Proc. Natl. Acad. Sci. USA 1989 ; 86, 2878), J.H. Richardson et al. (Science 1989 ; 246, 821), S.L. Hauser et al. (Nature 1986 ; 322, 176) et A. Karpas et al. (Nature 1986 ; 322, 177), à rechercher une implication de ce rétrovirus humain dans la SEP, cependant sans succès ou avec des résultats évoquant des réactions croisées.

Il existe par ailleurs un modèle animal très proche de la SEP, induit par un rétrovirus : le virus MAEDI-VISNA du mouton. Il est connu que l'infection naturelle par ce virus provoque une maladie ovine proche de la SEP, comme le rapportent Johnson R.T. (Rev. Infect. Dis. 1985 ; 7, 66-67), Narayan O. et Cork L.C. (Rev. Infect. Dis. 1985 ; 7, 89-98) et Nathanson N. et al. (Rev. Infect. Dis. 1985 ; 7, 75-82). L'infection expérimentale de moutons par inoculation intra-ventriculaire de souches neurovirulentes du virus VISNA a permis d'établir la responsabilité de ce virus dans la génèse de cette infection démyélinisante du mouton. Comme l'expliquent Nathanson N. et al. (Rev. Infect. Dis. 1985 ; 7, 75-82), Hoffman P.M. et Panitch H.S. ("Handbook of Clinical Neurology, 12 ; Viral diseases" R.R. Mc Kendall, ed., Elsevier Science Publishing, Amsterdam, 1989, 453-466) et A. Haase (Nature 1986 ; 322, 130-136), elle diffère quelque peu de l'infection naturelle, mais reste néanmoins proche de la SEP. Il est par ailleurs intéressant de noter que dans l'ensemble des travaux effectués sur ce sujet par les auteurs précités, le virus Visna est retrouvé dans les cellules de plexus choroïdes du cerveau des moutons infectés, qui constituent un site de latence et de réplication. occasionnelle du provirus Visna ; la localisation de ces cellules à l'interface sang/liquide céphalo-rachidien expliquant certainement ce phénomène.

Tous ces résultats plaident en faveur du rôle dans la SEP d'un rétrovirus inconnu.

Récemment, les travaux de H. PERRON et al. (Res. Virol. 1989 ; 140, 551-561 dans "Current concepts in multiple sclerosis" Wiethölter et al., eds. Amsterdam, Elsevier, 1991, p. 111-116 et The Lancet 1991 ; 337, 862-863) ont permis, à partir d'une ponction lombaire de liquide céphalo-rachidien d'un patient souffrant de SEP, d'isoler une lignée de cellules non lymphoïdes et de mettre en évidence la présence d'un virus, présentant les caractéristiques d'un rétrovirus et montrant en particulier une activité transcriptase inverse, dans le surnageant des cultures des cellules de cette lignée. L'étude au microscope électronique des cellules de cette lignée a permis de mettre en évidence des particules virales d'un diamètre compris environ entre 110 et 140 nm, la taille des particules variant selon qu'il s'agit de particules matures ou immatures. Par ailleurs, une étude sérologique, selon la technique ELISA, utilisant un extrait cellulaire des cellules infectées de cette lignée, a permis de montrer, avec 40 sérums de patients parmi lesquels 20 sont atteints de SEP (SEP certaine) et 20 sont des malades présumés (SEP probable), 60 % de résultats positifs. Une étude comparative avec 40 sérums de patients atteints d'autres maladies neurologiques que la SEP n'a donné que 5 % de résultats positifs. Cette lignée, que les auteurs ont dénommée LM7, est clonale et non-immortelle, et son étude immunocytochimique et ultrastructurale a permis de caractériser son origine leptoméningée.

Ce virus s'est avéré cependant très difficile à étudier, du fait que d'une part il s'exprime très faiblement, in vitro, dans la lignée cellulaire primaire d'origine leptoméningée, et que d'autre part cette lignée cellulaire dégénère assez rapidement après une trentaine de passages par extinction de son pouvoir mitotique, de sorte qu'elle ne permet plus l'expression virale.

Aussi, les auteurs ont envisagé une nouvelle approche (H. Perron et al., The Lancet, vol. 337, 862-863, (1991)), qui consiste à prélever un échantillon sanguin chez un patient atteint de SEP, à effectuer une culture de monocytes et à recueillir le surnageant pour vérifier l'expression d'une activité transcriptase inverse, soit directement dans le culot d'ultracentrifugation, soit après sédimentation à l'équilibre sur gradient de saccharose. Il a ainsi été montré un pic d'activité transcriptase inverse dans le surnageant chez les patients atteints de SEP et que cette activité est retrouvée dans la fraction de densité 1,17 g/ml environ. Une étude au microscope électronique des cellules infectées a permis de mettre en évidence des particules semblables à des rétrovirus de 100 à 120 nm, qui sont retrouvées dans les culots d'ultracentrifugation de surnageants de cultures exprimant une activité transcriptase inverse élevée. Cependant, comme expliqué par les auteurs, un effet cytopathique a été observé dans les cellules de sang de cordon infectées mais n'est plus détectable, de sorte que ce mode de culture n'est pas satisfaisant pour une étude approfondie des caractéristiques de ce virus. Les culots de centrifugation contenant des débris cellulaires et potentiellement des particules virales ont ensuite été cultivés sur des cellules du sang de cordon, pour la mise en évidence d'une expression virale.

Il est donc indispensable de disposer d'un procédé pour la culture in vitro de cellules infectées par un virus associé à la sclérose en plaques, un tel procédé n'étant pas disponible à ce jour.

Les inventeurs ont tout d'abord émis et vérifié l'hypothèse que des cellules de plexus choroïdes humains pourraient être des cellules permissives au virus rétrouvé chez des patients atteints de SEP. Sur la base de cette découverte des propriétés des cellules de plexus choroïdes humains, les présents inventeurs ont développé un procédé de culture in vitro de cellules infectées par un virus associé à la SEP et ont montré que ledit procédé permet l'obtention de lignées cellulaires infectées assurant une bonne réplication et expression du virus.

Le procédé selon l'invention consiste à :
* cultiver des cellules humaines infectées par une souche virale associée à la SEP, pour obtenir au moins une culture de cellules primaires infectées par ladite souche virale,
* cultiver des cellules humaines permissives, préférentiellement des cellules de plexus choroïdes humains non infectées, pouvant s'infecter et répliquer ladite souche virale pour obtenir au moins une culture permissive,
* cocultiver au moins un prélèvement d'une culture de cellules primaires infectées et un prélèvement d'une culture permissive, pour obtenir une première culture dérivée infectée par unedite souche virale,
* cultiver en série, c'est-à-dire par repiquages successifs, la première culture dérivée infectée ; à cette fin, on répète au cours du temps l'étape consistant à cocultiver, par exemple pendant 5 à 8 jours, un nouveau prélèvement d'une culture permisssive non infectée et un prélèvement de la première culture dérivée infectée, ou d'une sous-culture de cette dernière, pour obtenir une nouvelle sous-culture de la même première culture dérivée infectée, constituant une culture virale viable.

Aussi, selon l'invention, le procédé consiste tout d'abord à effectuer une culture de cellules de plexus choroïdes humains , dans un milieu de culture approprié, comprenant au moins des acides aminés, des facteurs vitaminiques, des sels inorganiques et du glucose, respectivement à des concentrations pondérales totales comprises entre 400 et 2250 mg/l, 3,5 et 130 mg/l, 9100 et 13000 mg/l, et 1000 et 6000 mg/l; puis à mettre en contact lesdites cellules de plexus choroïdes ainsi cultivées, dans leur milieu de culture, avec des cellules primaires infectées par le virus ou un surnageant de culture contenant le virus, ou avec des cellules dérivées desdites cellules infectées, dans des conditions déterminées permettant la propagation du virus des cellules infectées aux cellules cultivées, sa réplication et son expression.

Un milieu de culture bien adapté au procédé décrit précédemment comprend :
- entre 400 et 2250 mg/l d'acides aminés
- entre 3,5 et 130 mg/l de vitamines
- entre 9100 et 13000 mg/l de sels inorganiques
- entre 1000 et 6000 mg/l de glucose
- et éventuellement, au moins un facteur de croissance choisi parmi ECGF et FGF basique.

Plus particulièrement, et à titre d'exemple non limitatif, le milieu de culture ou de coculture comprend les constituants suivants:
* un ou plusieurs acides aminés choisis parmi les composés suivants, à savoir :
   - **arginine**: 100 à 500 mg/l, de préférence 100 à 300 mg/l
   - **cystéine et/ou cystine**: 25 à 300 mg/l, de préférence cystine: 25 à 100mg/l
   - **glutamine**: 200 à 1000 mg/l, de préférence 200 à 500 mg/l
   - **histidine** : 5 à 50 mg/l, de préférence 5 à 20 mg/l
   - **isoleucine** : 20 à 100 mg/l, de préférence 20 à 60 mg/l
   - **leucine** : 20 à 100 mg/l, de préférence 20 à 60 mg/l
   - **lysine** : 20 à 100 mg/l, de préférence 20 à 80 mg/l
   - **methionine** : 5 à 50 mg/l, de préférence 5 à 30 mg/l
   - **phénylalanine :** 10 à 70 mg/l, de préférence 10 à 50 mg/l
   - **thréonine** : 15 à 100 mg/l, de préférence 15 à 60 mg/l
   - **tryptophane** : 2 à 30 mg/l, de préférence 2 à 25 mg/l
   - **tyrosine** : 10 à 70 mg/l, de préférence 10 à 50 mg/l
   - **valine** : 10 à 80 mg/l, de préférence 10 à 60 mg/l
* une ou plusieurs vitamines choisies parmi les composés suivants :
   - **panthothenate** : 0,15 à 5 mg/l, de préférence sel de calcium : 0,15 à 2 mg/l
   - **choline** : 0,5 à 10 mg/l, de préférence sel de chlorure : 0,5 à 5 mg/l
   - **acide folique** : 0,5 à 10 mg/l, de préférence 0,5 à 5 mg/l
   - **inositol** : 1 à 70 mg/l, de préférence 1 à 50 mg/l
   - **nicotinamide et/ou niacinamide** : 0,5 à 10 mg/l, de préférence nicotinamide : 0,5 à 5 mg/l
   - **pyrridoxine et/ou pyrridoxal** : 0,5 à 10 mg/l, de préférence pyrridoxine-HCl : 0,5 à 5 mg/l
   - **riboflavine** : 0,05 à 1 mg/l, de préférence 0,05 à 0,5 mg/l
   - **thiamine** : 0,5 à 10 mg/l, de préférence 0,5 à 5 mg/l
* un ou plusieurs sels inorganiques choisis parmi les composés suivants, à savoir :
   - **sels de calcium** : 100 à 200 mg/l, de préférence CaCl2 anhydre
   - **chlorure de potassium** : 350 à 450 mg/l
   - **sels de magnésium** : 40 à 60 mg/l, de préférence MgSO₄ anhydre
   - **chlorure de sodium** : 6.000 à 8.000 mg/l
   - **sels de HCO**_{**3**} : 2.000 à 3.000 mg/l, de préférence NaHCO₃
   - **sels de HPO**_{**4**} : 600 à 1.000 mg/l, de préférence Na₂HPO₄ anhydre
   - et du **glucose** : 1.000 à 6.000 mg/l, de préférence D-glucose.

Avantageusement, les acides aminés sont choisis parmi ceux de la série naturelle L.

Le milieu peut également comprendre au moins un antibiotique, de préférence un mélange de pénicilline et de streptomycine, et si souhaité de la clindamycine pour empêcher les contaminations mycoplasmiques.

Selon un mode de réalisation de l'invention, le milieu comprend de plus au moins un facteur de croissance choisi parmi ECGF ("Endothelial Cell Growth Factor") dénommé également FGF acide, et FGF basique ("Fibroblast Growth Factor"), dans des proportions variables que l'homme du métier peut déterminer à partir de ses connaissances générales des cultures cellulaires et des produits qu'il a à sa disposition. A titre d'exemple, la concentration du facteur de croissance est comprise entre 1 et 50 µg par litre du milieu de culture, en présence d'héparine entre 50 et 150 µg/l. Avantageusement, le facteur de croissance choisi est ECGF à une concentration de 10 µg/l, en présence d'héparine comme précédemment.

Dans un mode de réalisation particulier de l'invention, les cellules permissives de plexus choroïdes humains avant mise en culture sont des cellules non infectées.

Selon un mode particulier de réalisation de l'invention, la culture des cellules primaires infectées, la première culture dérivée infectée, ou toute sous-culture de cette dernière, avant mise en contact avec les cellules permissives cultivées, sont préalablement traitées par irradiation, par exemple par irradiation par des rayons X.

Selon un mode préféré d'exécution de l'invention, on obtient plusieurs cultures de cellules primaires infectées par des souches virales ou isolats de SEP respectivement différents, et la première coculture est effectuée par coculture d'un prélèvement de la culture permissive et de plusieurs prélèvements de cultures de cellules primaires ou de sous-cultures respectivement différentes. On obtient ainsi dans la culture cellulaire viable un mélange de souches virales permettant une recombinaison inter-souches, la complémentation éventuelle de génomes défectifs et l'émergence de souches recombinées dont l'adaptabilité à certains critères peut être fortement accrue. Cela permet aussi d'obtenir des souches hautement adaptées à la culture in vitro, ou d'obtenir des souches réplicatives à partir de souches défectives.

Le terme "cellules infectées" tel qu'utilisé dans la présente invention fait référence:
i) à des cellules primaires infectées, obtenues à partir d'une culture de cellules directement issues d'un prélèvement de tissus ou de fluides biologiques in vivo ou post-mortem chez un individu infecté, ainsi qu'aux cellules dérivées obtenues par passages successifs de ces cellules primaires, et
ii) aux cellules secondaires infectées obtenues par co-culture de cellules primaires infectées et de cellules permissives, ainsi qu'aux cellules dérivées obtenues par passages de ces cellules secondaires.

Par "cellules primaires", on entend des cellules ou cultures provenant directement d'un prélèvement de tissus ou de fluides biologiques, et passées en culture sans aucune co-culture, ni aucune inoculation de souches virales issues d'autres cellules, pour autant que ces cellules dites primaires ne manifestent pas un potentiel de prolifération anormal (immortalisation ou transformation).

Les cellules infectées prélevées in vivo ou post-mortem peuvent être toutes cellules infectées par le virus, par exemple des cellules leptoméningées isolées du liquide céphalorachidien d'un patient (H. Perron et al., Res. Biol., 140, 551-561 (1989)), des cellules myéloïdes trouvées dans le sang, dans le liquide céphalorachidien, dans les tissus ou dans la moelle osseuse, en particulier des macrophages ou monocytes (H. Perron et al., The Lancet, vol. 337, 862-863, 10 avril 1991), et notamment des lymphocytes (S.A. Haahr et al., The Lancet, vol. 337, 863-864, 6 avril 1991) ou analogues.

Le terme "macrophage(s)" fait référence à des cellules dérivant directement de monocytes sanguins, à des cellules résidant dans les tissus (microgliocytes, cellules de Küpfer), et à des cellules du système réticulo endothélial, notamment les cellules de Langerhans.

Les cellules permissives sont des cellules qui peuvent s'infecter et permettre la replication d'un virus donné, avec production de particules virales extra-cellulaires que l'on peut étudier, notamment pour leur activité transcriptase inverse dans les surnageants.

Le terme "passage" refère à une culture cellulaire en série, et correspond à la dissociation de cellules d'un flacon de culture, pour les transférer dans un ou plusieurs nouveaux flacons.

Il est bien connu des spécialistes que des modifications spontanées ou induites peuvent survenir dans le caryotype pendant le stockage ou les passages. Ainsi des cellules dérivées d'une lignée cellulaire de référence peuvent ne pas être précisemment identiques aux cultures ou cellules d'origine. De même la variabilité génétique des rétrovirus est bien connue, et une souche rétrovirale donnée peut modifier ses caractéristiques par des mutations spontanées ou induites au cours des cultures.

L'invention sera mieux comprise à la lecture de la description détaillée qui va suivre, faite en référence aux figures annexées dans lesquelles :

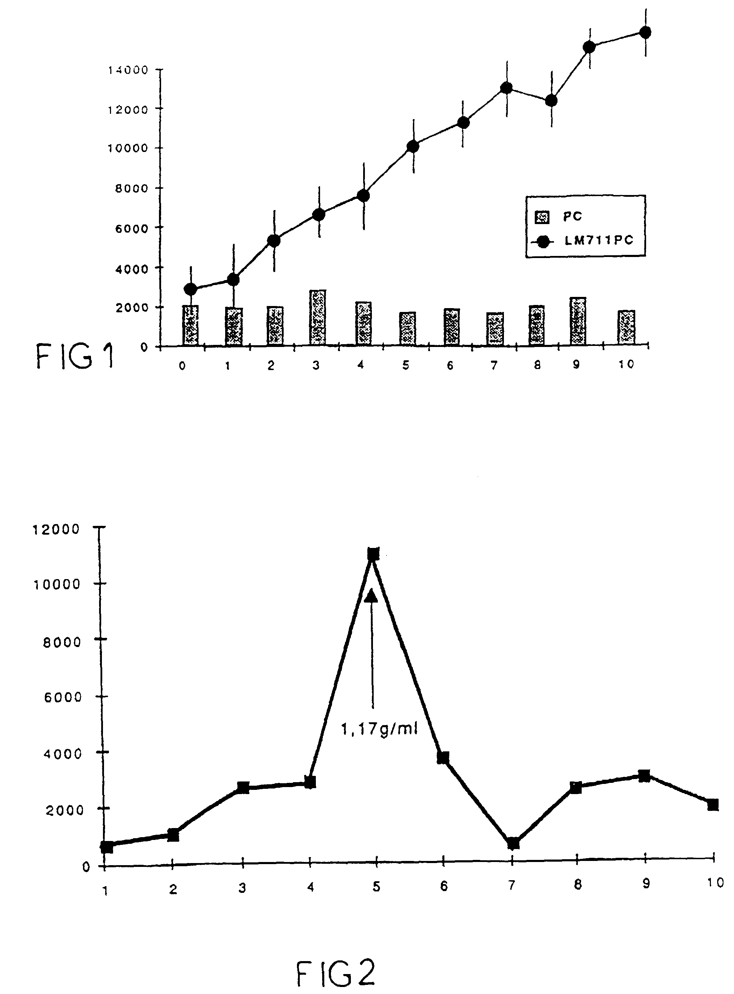

- la Figure 1, représente la cinétique de l'activité transcriptase inverse, en désintégrations par minute pour 10⁶ cellules en fonction du nombre de passages, détectée dans le surnageant d'une culture de cellules de plexus choroïdes humains référencées LM 711 PC, infectées par co-culture avec des cellules leptoméningées elles-mêmes infectées par le virus LM7, et comparaison avec l'activité transcriptase inverse détectée dans des cellules de plexus choroïdes humains non infectées, prélevées post-mortem chez un patient et n'ayant pas été co-cultivées;
- la Figure 2, représente l'activité transcriptase inverse de particules sédimentant à une densité connue pour les rétrovirus; en ordonnées est représenté le nombre de désintégrations par minute, testé sur 20 µl prélevés dans chaque fraction, et en abscisses est représentée la fraction par son numéro.

### Exemple 1 : Préparation in vitro d'une culture de cellules primaires infectées par un virus présent chez un patient atteint de SEP.

Les méthodes pour préparer des cultures primaires à partir de cellules primaires infectées, par exemple des cellules leptoméningées, des monocytes ou des lymphocytes, et les conditions pour leur croissance in vitro sont connues de l'homme du métier (voir références ci-dessus). Un autre candidat pour préparer une culture de cellules primaires infectées est représenté par les cellules de plexus choroïdes. Les cellules de plexus choroïdes sont mises en culture selon les techniques classiques, après explantation de plexus choroïdes humains obtenus post mortem. La pièce anatomique prélevée stérilement est délicatement dilacérée avec des pinces et placée dans une solution de trypsine pendant quelques minutes à environ 37°C. Les fragments tissulaires sont recueillis après centrifugation à basse vitesse (500 tpm) et le surnageant est recentrifugé à 1600 tpm pendant 5 à 10 minutes. Le culot est repris dans des milieux de culture et placé dans un flacon où seules les cellules adhérentes se maintiendront après changement du milieu.

### Exemple 2 : Préparation d'une culture de cellules permissives à un virus présent chez des patients atteints de SEP.

### Culture de cellules de plexus choroïdes

Des cellules de plexus choroïdes non infectées obtenues après explantation post mortem de plexus choroïdes humains sont cultivées dans un milieu RPMI 1640 (commercialisé par Boehringer Mannheim) comprenant : de la penicilline (200.000 U/l), de la streptomycine (200mg/l), de la clindamycine (75 mg/l), de la L-glutamine (6 mM/l), du pyruvate 1%, du sérum, de préférence du sérum de veau foetal 20 à 30% décomplémenté par incubation à 56°C pendant 30 minutes, et des acides aminés non essentiels 1% (Boehringer Mannheim MEM. A.A.N.E. 100x ref:210293). Avantageusement, le milieu de culture comprend de plus un facteur de croissance tel que le facteur de croissance de cellules endothéliales (ECGF) associé à de l'héparine (BOEHRINGER ref. 1/79/87 : ECGF environ 1 à 20ng/ml comprenant de l'héparine 50-150µg/ml).

### Exemple 3 : Coculture d'une lignée cellulaire primaire infectée par un virus présent chez un patient atteint de SEP et de cellules permissives au virus.

Des cellules d'une culture de cellules primaires infectées, comme décrit dans l'exemple 1 par un virus présent chez un individu atteint de SEP, par exemple le virus LM7, identifié dans le préambule de la présente description, sont dissociées de leur flacon de culture, les cellules viables adhérant au fond du flacon, puis reprises dans un milieu de culture approprié à la coculture, c'est-à-dire le milieu de culture des cellules de plexus choroïdes, selon l'exemple 2. Parallèlement, les cellules de plexus choroïde non infectées sont dissociées de leur milieu de culture décrit dans l'exemple 2, dans une solution de trypsine-EDTA. Les cellules sont ensuite centrifugées et resuspendues dans leur milieu de culture et sont rajoutées au flacon de la culture cellulaire infectée. Le flacon est placé dans une étuve à CO₂ et on laisse les cellules de plexus choroïdes adhérer et proliférer au fond du flacon, contenant déjà les cellules infectées, pendant 24 H. Le milieu est changé après 24 H et le mélange de cellules adhérentes est laissé dans l'étuve à CO₂, jusqu'à ce que la prolifération cellulaire produise une couche confluente, c'est-à-dire un tapis de cellules adhérentes. A ce stade, les cellules sont maintenues encore 5 à 7 jours pour assurer le transfert du virus des cellules infectées aux cellules de plexus choroïdes. La culture cellulaire est ensuite dédoublée et passée dans deux nouveaux flacons qui sont chacun réensemencés avec les cellules de plexus choroïdes dissociées en suspension. Ces nouvelles cultures sont soumises aux mêmes conditions que décrites ci-dessus pour l'adhésion et la prolifération des cellules, le transfert, l'expression et la réplication du virus. Les cultures de cellules sont ensuite dédoublées régulièrement et passées aussi longtemps que le potentiel mitotique des cellules permissives le permet. Ces cellules qui hébergent et produisent un virus du type LM7 peuvent à leur tour être utilisées pour infecter de nouvelles cellules par coculture comme décrit précédemment, et ainsi maintenir la souche virale en culture.

Les milieux de culture sont toujours changés au moins deux fois par semaine et toujours le lendemain d'un nouveau passage, c'est-à-dire à chaque nouvel ensemencement d'un flacon avec des cellules dissociées en suspension.

Préalablement à la coculture, les cellules hébergeant une souche virale peuvent éventuellement être irradiées de manière à éviter leur prolifération ultérieure au sein d'une culture nouvellement infectée. L'irradiation peut par exemple être réalisée avec une dose totale de 6.000 rad de rayons X.

Le suivi de la transmission d'un virus de type LM7 et du maintien de son expression dans les cellules obtenues après coculture avec des cellules produisant un tel virus, est réalisé par dosage de l'activité transcriptase inverse dans le surnageant des cultures, qui est régulièrement prélevé pour renouveler le milieu.

Le dosage de l'activité de cette enzyme caractéristique des rétrovirus se fait dans des conditions déterminées pour la souche LM7. Dans le cas du virus LM7, les conditions de réaction sont telles que décrites par H. Perron et al. (Res. Virol. 1989 ; 840, 551-561).

Les surnageants de culture, d'un volume minimal de 15 ml, sont collectés deux fois par semaine, précentrifugés à 10.000 tpm pendant 30 minutes pour éliminer les débris cellulaires, et ensuite ultracentrifugés à 35.000 tpm pendant 2 heures, pour sédimenter les particules rétrovirales. Les culots sont repris (volume final concentré environ 1000 fois dans un tampon Tris/HCl 0,05 M, pH 8 à 9,5) et conservés à - 80°C pour un dosage ultérieur de l'activité transcriptase inverse.

### Exemple 4 : Dosage de l'activité transcriptase inverse pour le suivi de la production de particules virales de type LM7 dans le surnageant des cellules de plexus choroïde nouvellement infectées.

Toutes les étapes sont effectuées avec du matériel et des solutions stériles, afin d'éviter toute interférence avec des nucléases ou protéases bactériennes, notamment pendant les phases d'incubation à 37°C.

Les culots contenant les particules virales concentrées sont décongelés et homogénéisés : 20µl sont prélevés et ajoutés dans un mélange réactionnel contenant : 5µl Tris 0.5 M-DTT 0,04 M pH 8.2/5µl NaCl 0.1 M/5µl MgCl2 0.3 M/23µl H₂O bidistillée /10µl NP4O 2%/ 2µl polyCm-oligodG12-18 (10 U O.D./ml ; Pharmacia) 5µl 3H-,3H-Guanosine-Tri-Phosphate (1 mCi/ml ; NEN). Les tubes en verre contenant les mélanges sont incubés à 37°C pendant 75 min. La réaction est arrêtée en ajoutant 75µl d'une solution à +4°C contenant : 12,5 % H₂O saturée avec du phosphate de sodium, 12,5 % H₂O saturée avec du pyrophosphate de sodium et 20 % d'acide Tri-Chloro-Acétique (TCA). Après 30 min. à 1 heure à 4°C, les tubes sont remplis avec une solution de 5% TCA, vidés et rincés 5 fois avec la solution à 5% de TCA, sur une membrane d'acetate de cellulose (Sartorius ref. 11106 25 N ; diamètre de pore : 0,45µ ; diamètre de la membrane : 25mm) à travers laquelle les échantillons sont filtrés dans un collecteur de fractions 1125 (Millipore ; ref. XX2702550). Avant d'être enlevées, les membranes sont rincées encore une fois avec 20 ml de TCA 5%. Les membranes sont alors placées dans des fioles que l'on remplit de liquide scintillant (Ready-Safe, Beckman) et l'activité est mesurée dans un compteur bêta, en cpm (coups par minute) et dpm (désintégrations par minute).

Chaque échantillon est testé en triple et la moyenne des valeurs utilisée comme résultat. Si la différence entre cette moyenne et l'une des mesures excède deux fois l'écart type mesuré sur des valeurs de référence, l'échantillon correspondant est testé à nouveau.

Ainsi, il a été possible de réaliser la cinétique de production de virions relargués dans le surnageant de cultures de cellules de plexus choroïde humain nouvellement infectées après coculture avec des cellules leptoméningées infectées par le virus LM7 (Figure 1).

Pour vérifier que l'activité transcriptase inverse est bien associée à des particules de type rétroviral, les culots de virion concentrés par ultracentrifugation de surnageants de culture sur coussin de glycérol sont placés sur des gradients de saccharose (15 à 50 % poids/poids) et ultracentrifugés à +4°C pendant 16 H à 100.000g dans un rotor à godets. 10 fractions sont recueillies et 20µl sont prélevés dans chaque fraction pour y doser l'activité transcriptase inverse comme décrit ci-dessus. Le pic spécifique d'activité est retrouvé dans la fraction de densité 1,17 g/ml environ (dosage réfractométrique), ce qui correspond à une densité de sédimentation à l'équilibre connue pour les particules rétrovirales (1,16 à 1,18 g/ml). Un exemple de ce dosage sur gradient est présenté dans la Figure 2.

## Revendications

1. Procédé d'obtention d'une culture ou lignée cellulaire infectée viable comprenant des cellules infectées par au moins une souche virale humaine associée à la sclérose en plaques (SEP), **caractérisé en ce qu'il** consiste en la combinaison des étapes suivantes :
**a)** on cultive des cellules humaines infectées par unedite souche virale, pour obtenir au moins une culture de cellules primaires infectées par ladite souche virale,
**b)** on cultive des cellules humaines non infectées et permissives à ladite souche virale, pour obtenir au moins une culture permissive,
**c)** on co-cultive au moins un prélèvement d'une culture de cellules primaires infectées et un prélèvement de la culture permissive, pour obtenir une première culture dérivée infectée par unedite souche virale,
**d)** on cultive en série la première culture dérivée infectée, et à cette fin on répète au cours du temps l'étape consistant à cocultiver un nouveau prélèvement d'une culture permissive non infectée et un prélèvement de la première culture dérivée infectée, ou d'une sous-culture de cette dernière, pour obtenir une nouvelle sous-culture de la même première culture dérivée infectée, constituant une culture virale viable.

2. Procédé selon la revendication 1, caractérisé en ce que la culture de cellules primaires infectées est obtenue à partir de cellules humaines infectées par ladite souche virale, choisies dans le groupe comprenant des cellules leptoméningées, des cellules de plexus choroïdes, des cellules myéloïdes sanguines, notamment des macrophages et monocytes, et des lymphocytes.

3. Procédé selon la revendication 1, caractérisé en ce que la culture permissive est obtenue à partir de cellules humaines de plexus-choroïde.

4. Procédé selon la revendication 1, caractérisé en ce qu'on obtient plusieurs cultures primaires infectées par des souches virales respectivement différentes, et l'étape (b) est effectuée par coculture d'un prélèvement de la culture permissive et de plusieurs prélèvements de cultures primaires ou de sous-cultures infectées respectivement différentes.

5. Procédé selon la revendication 1, caractérisé en ce que le milieu de culture, au moins pour la culture de cellules humaines permissives, comprend :
- entre 400 et 2250 mg/l d'acides aminés
- entre 3,5 et 130 mg/l de vitamines
- entre 9100 et 13000 mg/l de sels inorganiques
- entre 1000 et 6000 mg/l de glucose
- éventuellement, au moins un facteur de croissance choisi parmi ECGF et FGF basique.

## Patentansprüche

1. Verfahren zum Erhalten einer lebensfähigen, infizierten Zellkultur oder -linie mit Zellen, die mit wenigstens einem menschlichem Virus-Stamm infiziert sind, der mit Multipler Sklerose assoziiert ist, dadurch gekennzeichnet, daß es aus der Kombination der folgenden Schritte besteht:
a) man kultiviert menschliche Zellen, die mit einem derartigen Virus-Stamm infiziert sind, um wenigstens eine Kultur von primären Zellen zu erhalten, die mit dem genannten Virus-Stamm infiziert sind,
b) man kultiviert menschliche Zellen, die nicht infiziert sind und für den genannten Virus-Stamm permissiv sind, um wenigstens eine permissive Kultur zu erhalten,
c) man ko-kultiviert wenigstens eine Probe einer Kultur der infizierten primären Zellen und eine Probe der permissiven Kultur, um eine abgeleitete, primäre Kultur zu erhalten, die mit einem derartigen Virus-Stamm infiziert ist,
d) man kultiviert die infizierte, abgeleitete primäre Kultur in Serie und wiederholt zu diesem Zweck über der Zeit den Schritt der aus dem Ko-Kultivieren einer neuen Probe einer nicht infizierten permissiven Kultur und einer Probe der infizierten, abgeleiteten primären Kultur oder einer Subkultur der letzteren besteht, um eine neue Subkultur derselben infizierten, abgeleiteten primären Kultur zu erhalten, wodurch eine lebensfähige virale Kultur gebildet wird.

2. Verfahren nach Anspruch 1, dadurch gekennzeichnet, daß die Kultur der infizierten primären Zellen ausgehend von mit dem genannten Virus-Stamm infizierten menschlichen Zellen erhalten wird, die ausgewählt sind aus der Leptomeningitis-Zellen, Plexus Choroidei-Zellen, myeloide Blutzellen, insbesondere Makrophagen und Monozyten, sowie Lymphozyten umfassenden Gruppe.

3. Verfahren nach Anspruch 1, dadurch gekennzeichnet, daß die permissive Kultur ausgehend von menschlichen Plexus Choroidei-Zellen erhalten wird.

4. Verfahren nach Anspruch 1, dadurch gekennzeichnet, daß man mehrere mit jeweils verschiedenen Virus-Stämmen infizierte primäre Kulturen erhält und der Schritt (b) durchgeführt wird durch Ko-Kultivieren einer Probe der permissiven Kultur und mehreren Proben der primären Kulturen oder von jeweils unterschiedlich infizierten Subkulturen.

5. Verfahren nach Anspruch 1, dadurch gekennzeichnet, daß das Kulturmilieu wenigstens für die Kultur der permissiven menschlichen Zellen umfaßt:
- zwischen 400 und 2250 mg/l an Aminosäuren,
- zwischen 3,5 und 130 mg/l an Vitaminen,
- zwischen 9100 und 13000 mg/l an anorganischen Salzen,
- zwischen 1000 und 6000 mg/l an Glukose, und
- gegebenenfalls wenigstens einen Wachstumsfaktor ausgewählt unter ECGF und basischem FGF.

## Claims

1. Process for the production of a viable infected culture or cell line comprising cells infected by at least one human viral strain associated with multiple sclerosis (MS), characterized in that it comprises the combination of the following stages:
a) human cells infected by a said viral strain are cultivated to obtain at least one culture of primary cells infected by the said viral strain,
b) non-infected human cells which are permissive to the said viral strain are cultivated to obtain at least one permissive culture,
c) at least one sample of a culture of infected primary cells and one sample of the permissive culture are cocultured to obtain a first derived culture infected by a said viral strain,
d) the first infected derived culture is cultivated in series, and for this purpose the stage comprising cocultivation of a new sample of a non-infected permissive culture and a sample of the first infected derived culture, or of a subculture of the latter, is repeated in the course of time to obtain a new subculture of the same first infected derived culture, constituting a viable viral culture.

2. Process according to Claim 1, characterized in that the culture of infected primary cells is obtained from human cells infected by the said viral strain chosen from the group comprising leptomeningeal cells, plexus choroideus cells, myeloid blood cells, in particular macrophages and monocytes, and lymphocytes.

3. Process according to Claim 1, characterized in that the permissive culture is obtained from human plexus choroideus cells.

4. Process according to Claim 1, characterized in that several primary cultures infected by viral strains which differ respectively are obtained, and stage (b) is carried out by coculture of a sample of the permissive culture and of several samples of infected primary cultures or subcultures which differ respectively.

5. Process according to Claim 1, characterized in that the culture medium, at least for culture of permissive human cells, comprises:
- between 400 and 2250 mg/l of amino acids
- between 3.5 and 130 mg/l of vitamins
- between 9100 and 13,000 mg/l of inorganic salts
- between 1000 and 6000 mg/l of glucose
- if appropriate, at least one growth factor chosen from ECGF and basic FGF.
